# EUROPEAN PATENT APPLICATION

(11) **EP 2 128 611 A1**
(43) Date of publication of application: **02.12.2009**
(21) Application number: 08721615.6
(22) Date of filing: 07.03.2008
(51) Int. Cl.: G01N 33/14, C12N 15/09, C12Q 1/68, G01N 33/53

(54) **METHOD OF EVALUATING FOAM-HOLDING PROPERTIES OF FERMENTED MALT DRINK AND MARKER FOR EVALUATING FOAM-HOLDING PROPERTIES**

(30) Priority: 07.03.2007 JP 2007057632; 07.03.2007 JP 2007057724
(71) Applicant: Sapporo Breweries Limited, Tokyo 150-8522 (JP)
(72) Inventor: IIMURE, Takashi, Tokyo 150-8522 (JP); ITO, Kazutoshi, Tokyo 150-8522 (JP); TAKEDA, Kazuyoshi, Kurashiki-shi Okayama 710-0031 (JP)
(74) Representative: Vossius & Partner
(86) International application number: PCT/JP2008/054198
(87) International publication number: WO 2008/108470

(57) **Abstract**

The present invention provides a method for determination of the foam stability of a fermented malt beverage, wherein the concentration of protein Z7 (M_{Z7}) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, is used as an index having a negative correlation with the foam stability of the fermented malt beverage, to determine the foam stability of the fermented malt beverage. According to the determination method of the invention, it is possible to determine the foam stability from the concentration of a prescribed protein in the barley seeds, malt, wort, fermented malt beverage or the like without directly measuring the head retention of the fermented malt beverage.

## Description

### Technical Field

The present invention relates to a method for determination of the foam stability of a fermented malt beverage and to a marker for determination of foam stability. The present invention further relates to a barley selection method and barley selection marker.

### Background Art

Foam on fermented malt beverages such as beer is an aggregate of carbon dioxide gas bubbles surrounded by thin liquid films. It acts as a lid to prevent loss of carbonation and alteration of flavor, while also enhancing the aroma. The foam stability of fermented malt beverages is therefore one of the important factors for judging the quality of fermented malt beverages, and research has been conducted to improve the foam stability of fermented malt beverages.

The foam stability of a fermented malt beverage has been determined based on the NIBEM value, i.e. the time (seconds) required for the foam to collapse over a distance of 30 mm after the fermented malt beverage at 20°C has been poured into a standard glass with a foam dispenser.

Also as for barley to be used as a material, since the foam stability of a fermented malt beverage is one of the important factors for judging the quality of the fermented malt beverage, there is demand for a barley that improves the foam stability of a fermented malt beverage.

Examples of barley selection methods include: a method wherein a hybrid produced by crossing elite lines is cultivated until a desired trait is expressed, and a method wherein in the seed or seedling stage, genomic DNA and proteins of a hybrid are examined to see if a DNA region or protein known to be a selection marker is present.

LTP-1 and hordein have been reported as barley proteins associated with the foam stability of fermented malt beverages (Non-patent documents 1 and 2).

Recently, proteomics has come to be used for selection of plants, and attempts have been made to comprehensively analyze the protein compositions of barley seeds based on genomic information, and to identify proteins associated with malt qualities by comparing the malt qualities with two-dimensional gel electrophoresis patterns (Non-patent documents 3 to 5).
Non-patent document 1: Bamforth et al., 2004, J. Sci. Food Agric., Vol. 84, p.1001-1004
Non-patent document 2: Van Nierop et al., 2004, J. Agric. Food Chem., Vol. 52, p.3120-3129
Non-patent document 3: Ostergaard et al., 2004, Proteomics, Vol. 4, p.2437-2447
Non-patent document 4: Sass Bak-Jensen et al., 2004, Proteomics, Vol. 4, p.728-742
Non-patent document 5: Finnie and Svensson, 2004, Proc. 9th International Barley Genetics Symposium, p.431-436

### Disclosure of the Invention

### Problem to be Solved by the Invention

The NIBEM value, which is used as a criterion for determining the foam stability of a fermented malt beverage, is greatly affected by temperature, weather and artificial factors, and it is difficult to determine the foam stability by comparing NIBEM values measured under different conditions.

Also, although LTP-1 and hordein in barley have been reported to be associated with the foam stability of a fermented malt beverage, the correlation with the NIBEM value has not been elucidated, and these have not been used as generally applicable markers for determination of foam stability.

In addition, although proteomic studies of barley have been promoted, no new proteins associated with the foam stability of a fermented malt beverage have been discovered.

As for barley selection methods as well, there have been found no selection markers that would allow selection of a barley that improves the foam stability of a fermented malt beverage, and there have been no selection methods for a barley that improves the foam stability of a fermented malt beverage.

As mentioned above, although LTP-1 and hordein in barley have been reported to be associated with the foam stability of a fermented malt beverage, the correlation with the NIBEM value has not been elucidated. It is therefore difficult to use them as a selection marker for a barley to be used in the production of a fermented malt beverage with improved foam stability.

It is an object of the present invention to provide a method for determination of the foam stability of a fermented malt beverage, which makes it possible to determine the foam stability from the concentration of a prescribed protein in the barley seeds, malt, wort, fermented malt beverage or the like without directly measuring the head retention of the fermented malt beverage. It is another object of the present invention to provide a marker for determination of the foam stability or a fermented malt beverage.

It is yet another object of the present invention to provide a selection method for a barley that improves the foam stability of a fermented malt beverage. It is yet another object of the present invention to provide a selection marker for a barley that improves the foam stability of a fermented malt beverage.

### Means for Solving the Problem

In order to achieve the objects stated above, the present invention provides a method for determination of the foam stability of a fermented malt beverage, wherein the concentration of protein Z7 (M₂₇) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, is used as an index having a negative correlation with the foam stability of the fermented malt beverage, to determine the foam stability of the fermented malt beverage.

The present inventors analyzed proteins in beer using proteomics as a tool, and found that in the case of a beer with high NIBEM value and good head retention, the protein Z7 concentration is low. Also, as a result of statistical analysis of the correlation between the protein Z7 concentration and NIBEM value, the present inventors found that the protein Z7 concentration can be used for determination of the foam stability of a fermented malt beverage.

In the determination method defined above, measurement of the concentration of protein Z7 in the barley seeds, malt, wort, fermented malt beverage or pre-fermentation or fermenting material solution of the fermented malt beverage makes it possible to determine the foam stability without measuring the NIBEM value. Since measurement of the protein Z7 concentration can be performed without the effects of temperature, weather and artificial factors, it allows more generally applicable and more accurate determination of the foam stability than measurement of the NIBEM value.

In the determination method defined above, it is preferred, for example, that the value of the formula: a - b × M_{Z7} [where a is a positive or negative number or 0, and b is a positive number] be used as an index having a positive correlation with the foam stability of the fermented malt beverage, to determine the foam stability of the fermented malt beverage. The value can be used as a value having a linear relationship with the NIBEM value, to determine the foam stability of the fermented malt beverage. If a is 0, the determination can be performed more conveniently.

Also, it is preferred that a and b in the formula: a - b × M_{Z7} be the simple regression coefficients of a predictive simple regression equation for the NIBEM value of the fermented malt beverage as a function of M_{Z7}.

In this case, the NIBEM value can be predicted from the protein Z7 concentration (M_{Z7}) without directly measuring the NIBEM value using a specialized apparatus, standard glass, etc. for measuring the NIBEM value. The foam stability measured by this determination method can be compared to the NIBEM value that has already been measured by a conventional method.

The present invention also provides a marker for determination of the foam stability of a fermented malt beverage, the marker being composed of protein Z7.

Protein Z7 can be considered a negative factor for the foam stability of a fermented malt beverage. Since a high concentration of protein Z7 in a fermented malt beverage or a pre-fermentation or fermenting material solution of a fermented malt beverage impairs the foam stability of the fermented malt beverage, it can be used as a marker for determination of foam stability.

The present invention further provides a method for selection of a barley that improves the foam stability of a fermented malt beverage, wherein protein Z7 is used as a selection marker, the method comprising: a measurement step in which the concentration of protein Z7 in seeds of a barley, a malt obtained from the seeds, a wort obtained from the malt, a fermented malt beverage obtained by fermenting the wort, or a pre-fermentation or fermenting material solution of the fermented malt beverage, is measured, and a judgment step in which, if the concentration is lower than a prescribed reference value, the barley is judged to be a barley that improves the foam stability of a fermented malt beverage.

The present inventors comprehensively analyzed proteins in beer using proteomics as a tool, and found that in the case of a beer with high NIBEM value and good head retention, the concentration of protein Z7 in seeds of a barley which is used as a material or in wort, beer, etc. is low. Also, as a result of statistical analysis of the correlation between the protein Z7 concentration and NIBEM value, the present inventors found that examination of the protein Z7 concentration makes it possible to determine and predict the foam stability of the fermented malt beverage.

Thus, if the concentration of protein Z7 in the barley seeds, malt, wort, fermented malt beverage or pre-fermentation or fermenting material solution of the fermented malt beverage is measured, it is possible to select a barley that improves the foam stability of a fermented malt beverage, without directly measuring the NIBEM value of a fermented malt beverage obtained as the final product. Also, the NIBEM value is greatly affected by temperature, weather and artificial factors, and it is difficult to determine the foam stability by comparing NIBEM values measured under different conditions. On the other hand, since the protein Z7 concentration can be measured without the effects of these factors and there is a correlation between the protein Z7 concentration and NIBEM value, it can be used for determination of the foam stability.

The measurement step preferably comprises: an extraction step in which protein is extracted from seeds of the barley, a malt obtained from the seeds, a wort obtained from the malt, a fermented malt beverage obtained by fermenting the wort, or a pre-fermentation or fermenting material solution of the fermented malt beverage, and a quantitation step in which an anti-protein Z7 antibody is used to quantitate protein Z7 in the protein.

Protein Z7 is specifically recognized by an anti-protein Z7 antibody, and therefore it can be detected with high sensitivity using, for example, ELISA, Western blotting or protein chip technology, thus allowing its concentration to be conveniently measured.

The present invention further provides a method for selection of a barley that improves the foam stability of a fermented malt beverage, wherein protein Z7 is used as a selection marker, the method comprising: an extraction step in which RNA is extracted from seeds of a barley or a malt obtained from the seeds, a quantitation step in which protein Z7 mRNA expressed in the seeds or malt is quantitated, and a judgment step in which, if the protein Z7 mRNA expression level is lower than a prescribed reference value, the barley is judged to be a barley that improves the foam stability of a fermented malt beverage.

Even when an anti-protein Z7 antibody, which specifically recognizes protein Z7, is not available, protein Z7 mRNA can be detected with high sensitivity using, for example, PCR, Northern blotting or DNA chip technology, thus allowing its expression level to be conveniently measured.

The present invention further provides a marker for selection of a barley that improves the foam stability of a fermented malt beverage, the marker being composed of protein Z7.

Protein Z7 can be considered a negative factor for the foam stability of a fermented malt beverage. Since a fermented malt beverage produced from seeds of a barley with low protein Z7 concentration has good head retention, the selection marker defined above can be utilized for selection of a barley variety suitable for use as a material for a fermented malt beverage with good head retention.

### Effects of the Invention

According to the present invention, it is possible to determine the foam stability and also predict the NIBEM value without directly measuring the NIBEM value. Also, the method for determination of foam stability according to the invention can be carried out without the effects of temperature, weather and artificial factors, and therefore it allows more generally applicable and more accurate determination of the foam stability than measurement of the NIBEM value.

According to the present invention, it is further possible to select a barley that improves the foam stability of a fermented malt beverage, without directly measuring the NIBEM value of a fermented malt beverage obtained as the final product using the barley as a material. Also, since measurement of the protein Z7 concentration can be performed without the effects of temperature, weather and artificial factors and there is a correlation between the protein Z7 concentration and NIBEM value, the selection method of the invention can be used as a screening method for a fermented malt beverage with good head retention.

In addition, the selection marker of the invention can be used for selection of a barley suitable for use as a material for a fermented malt beverage with good head retention.

### Brief Description of the Drawings

Fig. 1 is a graph showing the result of simple regression analysis performed using the NIBEM value as the response variable and the protein Z7 concentration in barley seeds as the explanatory variable.
Fig. 2 is a graph showing the result of simple regression analysis performed using the NIBEM value as the response variable and the protein Z7 concentration in Congress wort as the explanatory variable.
Fig. 3 is a graph showing the result of simple regression analysis performed using the NIBEM value as the response variable and the protein Z7 concentration in beer as the explanatory variable.

### Best Modes for Carrying Out the Invention

Preferred embodiments of the present invention will now be described in detail.

### [Method for determination of foam stability]

The determination method of the invention is a method for determination of the foam stability of a fermented malt beverage, wherein the concentration of protein Z7 (M_{Z7}) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, is used as an index having a negative correlation with the foam stability of the fermented malt beverage, to determine the foam stability of the fermented malt beverage.

As used herein, "head retention" refers to the time required for disappearance of the foam produced when pouring a fermented malt beverage into a glass. If the required time is long, the "head retention" is "good". If the required time is short, the "head retention" is "poor". "Foam stability" means the extent to which the head retention is good or poor, and it is a basis for comparing the head retention of a fermented malt beverage with that of another fermented malt beverage. A "method for determination of foam stability" is a method for determining whether the head retention of a fermented malt beverage is good or poor compared to another fermented malt beverage.

A "fermented malt beverage" is a beverage brewed using malt as a material, and as examples there may be mentioned beer, low-malt beer (happoshu), and other types of alcoholic beverages obtained using malt as a material. Beer is a fermented beverage obtained using malt, hops and water as materials or using malt, hops, water, and barley or another commodity established by the Japanese government ordinance (barley, rice, corn, kaoliang, potato, starch, saccharide, or a bittering agent or coloring agent approved by the Department of the Treasury) as materials, with the proportion of malt used being 2/3 or greater. Low-malt beer (happoshu) is an effervescent alcoholic beverage obtained using malt or barley as part of the materials, with the proportion of malt used being less than 2/3.

A "pre-fermentation material solution of a fermented malt beverage" is: the material solution that exists from the time the malt is subjected to mashing until wort is produced from the malt; the wort that exists before boiling; the wort that exists from the time the wort is boiled until it is cooled to yield cold wort to which yeast is to be added; or the cold wort. A "fermenting material solution of a fermented malt beverage" is the fermentate that exists from the time yeast is added to the cold wort, which is then subjected to fermentation, until a fermented malt beverage is obtained.

"Protein Z7" is a serine protease inhibitor present in barley seeds and beer, and it is a protein with a molecular weight of approximately 43 kDa (NCBI Accession CAA64599). In addition to protein Z7, barley seeds contain protein Z4 (NCBI Accession CAA66232), which belongs to the same subfamily of barley serine protease inhibitor, and its amino acid sequence has approximately 73% homology with protein Z7.

The concentration of protein Z7 (M_{Z7}) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, can be measured using, for example, ELISA, Western blotting, protein chip technology or affinity chromatography-based quantitative analysis.

The anti-protein Z7 antibody to be used in ELISA or Western blotting may be any antibody that can specifically recognize protein Z7 from a barley to be used for fermentation. For example, it can be prepared by immunizing a rabbit, mouse or the like using as antigen a peptide synthesized based on the amino acid sequence of protein Z7. These immunological methods are well known to those skilled in the art and will not be described in detail in the present specification. As for details of such methods, refer to "Antibodies: A Laboratory Manual" (Harlow and Lane, 1988, Cold Spring Harbor Laboratory), for example.

In the determination method defined above, it is preferred, for example, that the value of the formula: a - b × M_{Z7} [where a is a positive or negative number or 0, and b is a positive number] be used as an index having a positive correlation with the foam stability of the fermented malt beverage, to determine the foam stability of the fermented malt beverage. The value can be used as a value having a linear relationship with the NIBEM value, to determine the foam stability of the fermented malt beverage. If a is 0, the determination can be performed more conveniently.

Also, it is preferred that a and b in the formula: a - b × M_{Z7} be the simple regression coefficients of a predictive simple regression equation for the NIBEM value of the fermented malt beverage as a function of M_{Z7}. The simple regression coefficients of a predictive simple regression equation for the NIBEM value of a fermented malt beverage can be obtained by: measuring the concentration of protein Z7 (M_{Z7}) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, for a plurality of fermented malt beverages whose NIBEM values have been measured, and then performing simple regression analysis using the NIBEM value of the fermented malt beverage as the response variable and the protein Z7 concentration (M_{Z7}) as the explanatory variable.

The marker for determination of the foam stability of a fermented malt beverage is characterized by being composed of protein Z7.

Protein Z7 can be considered a negative factor for the foam stability of a fermented malt beverage. Since a low concentration of protein Z7 in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage improves the foam stability of the fermented malt beverage, it can be used as a marker for determination of foam stability. It has never been known that protein Z7 can be utilized for the determination or prediction of the foam stability of a fermented malt beverage.

Here, a "marker for determination of foam stability" is a protein which can serve as an index for determining or predicting the foam stability of a fermented malt beverage, wherein the concentration of the protein in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, shows a correlation with the NIBEM value.

Protein Z7 is present in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, and it can be quantitated using, for example, ELISA, Western blotting, protein chip technology or affinity chromatography-based quantitative analysis.

### [Barley selection method]

The selection method of the invention is a method for selection of a barley that improves the foam stability of a fermented malt beverage, wherein protein Z7 is used as a selection marker, the method comprising: a measurement step in which the concentration of protein Z7 in seeds of a barley, a malt obtained from the seeds, a wort obtained from the malt, a fennented malt beverage obtained by fermenting the wort, or a pre-fermentation or fermenting material solution of the fermented malt beverage, is measured, and a judgment step in which, if the concentration is lower than a prescribed reference value, the barley is judged to be a barley that improves the foam stability of a fermented malt beverage.

As used herein, a "barley" is a plant whose scientific name is *Hordeum vulgare,* and it may be a variety, line or individual.

"Head retention" refers to the time required for disappearance of the foam produced when pouring a fermented malt beverage into a glass. If the required time is long, the "head retention" is "good". If the required time is short, the "head retention" is "poor". A "barley that improves foam stability" is a barley that provides improved foam stability to a fermented malt beverage produced using the barley as a material, compared to another fermented malt beverage produced using the same materials except for barley.

A "fermented malt beverage" is a beverage brewed using malt as a material, and as examples there may be mentioned beer, low-malt beer (happoshu), and other types of alcoholic beverages obtained using malt as a material. Beer is a fermented beverage obtained using malt, hops and water as materials or using malt, hops, water, and barley or another commodity established by the Japanese government ordinance (barley, rice, corn, kaoliang, potato, starch, saccharide, or a bittering agent or coloring agent approved by the Department of the Treasury) as materials, with the proportion of malt used being 2/3 or greater. Low-malt beer (happoshu) is an effervescent alcoholic beverage obtained using malt or barley as part of the materials, with the proportion of malt used being less than 2/3.

A "pre-fermentation material solution of a fermented malt beverage" is: the material solution that exists from the time the malt is subjected to mashing until wort is produced from the malt; the wort that exists before boiling; the wort that exists from the time the wort is boiled until it is cooled to yield cold wort to which yeast is to be added; or the cold wort. A "fermenting material solution of a fermented malt beverage" is the fermentate that exists from the time yeast is added to the cold wort, which is then subjected to fermentation, until a fermented malt beverage is obtained.

"Protein Z7" is a serine protease inhibitor present in barley seeds and beer, and it is a protein with a molecular weight of approximately 43 kDa (NCBI Accession CAA64599). In addition to protein Z7, barley seeds contain protein Z4 (NCBI Accession CAA66232), which belongs to the same subfamily of barley serine protease inhibitor, and its amino acid sequence has approximately 73% homology to protein Z7.

The "prescribed reference value" recited above can be appropriately set depending on, for example, the purpose for which, and the conditions under which, the determination method is to be used, by analyzing the head retention (for example, NIBEM value) and protein Z7 concentration for different fermented malt beverages.

The measurement step defined above preferably comprises: an extraction step in which protein is extracted from seeds of the barley, a malt obtained from the seeds, a wort obtained from the malt, a fermented malt beverage obtained by fermenting the wort, or a pre-fermentation or fermenting material solution of the fermented malt beverage, and a quantitation step in which an anti-protein Z7 antibody is used to quantitate protein Z7 in the protein. The quantitation of protein Z7 can be performed using, for example, ELISA, Western blotting, protein chip technology or affinity chromatography-based quantitative analysis.

The anti-protein Z7 antibody to be used in ELISA or Western blotting may be any antibody that can specifically recognize protein Z7 from a barley to be used for fermentation. For example, it can be prepared by immunizing a rabbit, mouse or the like using as antigen a peptide synthesized based on the amino acid sequence of protein Z7. These immunological methods are well known to those skilled in the art and will not be described in detail in the present specification. As for details of such methods, refer to "Antibodies: A Laboratory Manual" (Harlow and Lane, 1988, Cold Spring Harbor Laboratory), for example.

The selection method of the invention is a method for selection of a barley capable of producing a fermented malt beverage with improved foam stability, wherein protein Z7 is used as a selection marker, the method comprising: an extraction step in which RNA is extracted from seeds of a barley or a malt obtained from the seeds, a quantitation step in which protein Z7 mRNA expressed in the seeds or malt is quantitated, and a judgment step in which, if the protein Z7 mRNA expression level is lower than a prescribed reference value, the barley is judged to be a barley capable of producing a fermented malt beverage with improved foam stability.

In the extraction step defined above, RNA can be extracted by, for example, the hot phenol method (method wherein RNA is extracted while dissolving barley seeds or malt with hot phenol and sodium dodecyl sulfate). However, in terms of obtaining high purity RNA with low degradation, it is preferred to freeze barley seeds or malt with liquid nitrogen, then grind it with a grinder and extract RNA with an RNA extraction reagent containing guanidine thiocyanate. The RNA extraction reagent may be, for example, Isogen^{™} (Nippon Gene).

In the quantitation step defined above, protein Z7 mRNA in the barley-derived RNA extracted in the extraction step can be detected with high sensitivity using, for example, RT-PCR, Northern blotting or DNA chip technology, thus allowing its expression level to be measured. Such methods are described in a genetic engineering protocol outlined in, for example, "Molecular Cloning" (Maniatis et al., 1989, Cold Spring Harbor Laboratory Press) or "Current Protocols in Molecular Biology" (F. M. Ausubel, 1988, John Wiley & Sons, Inc.).

The "prescribed reference value" recited above can be appropriately set depending on, for example, the purpose for which, and the conditions under which, the determination method is to be used, by analyzing the head retention (for example, NIBEM value) and protein Z7 mRNA expression level for different fermented malt beverages.

The marker for selection of a barley that improves the foam stability of a fermented malt beverage is characterized by being composed of protein Z7.

Protein Z7 can be considered a negative factor for the foam stability of a fermented malt beverage. Since a low concentration of protein Z7 improves the foam stability of the fermented malt beverage, it can be used as a marker for determination of foam stability and as a marker for selection of a barley suitable for use as a material for a fermented malt beverage with good head retention. It has never been known that protein Z7 can be utilized for the determination of the foam stability of a fermented malt beverage or the selection of a barley suitable for use as a material for a fermented malt beverage with good head retention.

Here, a "marker for determination of foam stability" is a protein which can serve as an index for determining or predicting the foam stability of a fermented malt beverage, wherein the concentration of the protein in the barley seeds, malt, wort, fermented malt beverage or pre-fermentation or fermenting material solution of the fermented malt beverage shows a correlation with the NIBEM value.

Protein Z7 is present in the barley seeds, malt, wort, fermented malt beverage or pre-fermentation or fermenting material solution of the fermented malt beverage, and it can be quantitated using, for example, ELISA, Western blotting, protein chip technology or affinity chromatography-based quantitative analysis.

### Examples

The present invention will now be explained in greater detail based on examples. However, the present invention is not limited to the examples described below.

### [Example 1: Correlation between the protein Z7 concentration in seeds of a barley to be used as a material for beer and the NIBEM value]

Malts were produced from seeds of 7 barley varieties (variety A: Haruna Nijo; variety B: CDC Kendall; variety C: Amagi Nijo; variety D: CDC Copeland; variety E: Hokuiku 39; variety F: Ryofu; variety G: Lofty Nijo), and 7 beers were produced using the same materials except for malt. The protein Z7 concentrations in seeds of the 7 barley varieties used as a material for beer and the NIBEM values of the 7 beers were measured, and the correlation between the protein Z7 concentration in barley seeds and NIBEM value was examined.

The protein Z7 concentrations in seeds of the 7 barley varieties were measured as follows. First, ground barley seeds (50 mg) were placed in a 2 mL tube equipped with a screw cap, and 1 mL of PBS (phosphate-buffered saline) containing 0.28% dithiothreitol (Wako) was added to yield a suspension, which was shaken overnight at 4°C to extract protein Z7. Next, the suspension was centrifuged at 15,000 rpm for 5 minutes at 4°C, and the obtained supernatant was appropriately diluted. Then, the protein Z7 was quantitated with an anti-protein Z7 antibody. The anti-protein Z7 antibody was provided by Dr. Evans of the University of Tasmania, Australia, and the quantitation of protein Z7 using the anti-protein Z7 antibody was performed according to an article by Dr. Evans (Amer. Soc. Brew. Chem., 2003, Vol. 61, p.55-62).

The beer brewing using seeds of the barley varieties was carried out as follows. First, seeds of the 7 barley varieties (varieties A to G) were soaked in water in a steep tank to a moisture content of about 43%, and after germination for 6 days in a germination room, they were dried with hot air to produce malts of the 7 barley varieties.

Next, corn starch, corn grits and rice, which were adjuncts, were added to each malt, to which water was subsequently added. Then, saccharification was performed at 50 to 60°C for 20 minutes, at 65°C for 40 minutes and at 73°C for 3 minutes (saccharification step). Hops were added to the obtained wort, and the mixture was boiled for 90 minutes. After cooling to 10°C, yeast was added and alcoholic fermentation was performed at 10 to 15°C for 7 to 10 days (fermentation step). After completion of the fermentation, the mixture was aged for 2 to 3 weeks at -1 to 2°C, and the obtained fermentate, which could be treated as beer, was subjected to measurement of the NIBEM value.

The measurement of the NIBEM value was performed using NIBEM-T, Inpack 2000 and a standard glass for measuring the NIBEM value (Haffmans). Specifically, each of the fermented malt beverages was brought to 20°C and poured into a standard glass with a foam dispenser using carbon dioxide gas. The collapse of the produced foam was followed using the NIBEM-T meter, and the time (seconds) required for the foam to collapse over a distance of 30 mm was recorded as the NIBEM value.

Table 1 shows the protein Z7 concentrations in the seeds of the 7 barley varieties (varieties A to G) and the NIBEM values of the 7 beers brewed using the seeds of the 7 barley varieties as a material.

**[Table 1]**

| Barley variety | Protein Z7 concentration in barley seeds (ng/mg seed) | NIBEM value (sec) |
|---|---|---|
| Variety A | 358.5 | 234 |
| Variety B | 102.5 | 265 |
| Variety C | 247.5 | 243 |
| Variety D | 200.0 | 247 |
| Variety E | 367.4 | 226 |
| Variety F | 222.6 | 251 |
| Variety G | 232.5 | 229 |

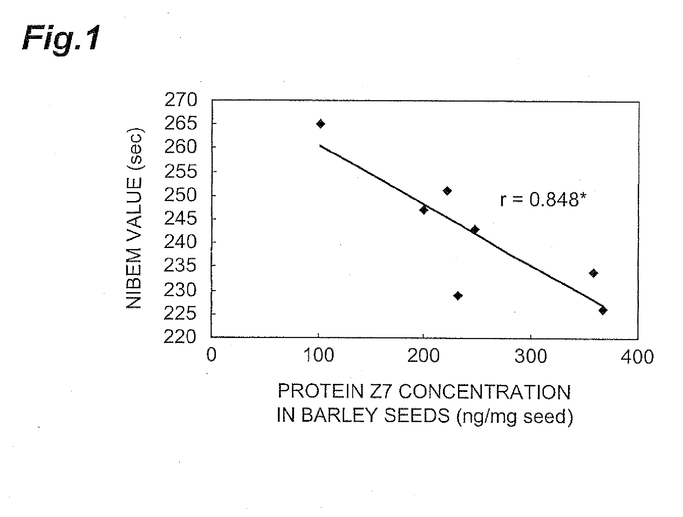

Fig. 1 is a graph showing the result of simple regression analysis performed using the NIBEM value as the response variable and the protein Z7 concentration in barley seeds as the explanatory variable. A single asterisk in the graph indicates significance at the 5% level.

A statistically significant correlation was found between the NIBEM value and protein Z7 concentration in barley seeds. The simple regression equation was: NIBEM value = 273.31 + (-0.126 × protein Z7 concentration) (r = 0.848).

The obtained results demonstrate that the protein Z7 concentration in barley seeds can be used as an index having a negative correlation with the foam stability of a fermented malt beverage produced using the barley seeds as a material, to determine the foam stability of the fermented malt beverage, and that measurement of the protein Z7 concentration in barley seeds allows estimation of the NIBEM value of the fermented malt beverage. Also, the results demonstrate that measurement of the protein Z7 concentration in barley seeds allows selection of a barley that improves the foam stability of a fermented malt beverage.

### [Example 2: Correlation between the protein Z7 concentration in Congress wort and the NIBEM value]

Worts were produced from seeds of 7 barley varieties (variety A: Haruna Nijo; variety B: CDC Kendall; variety C: Amagi Nijo; variety D: CDC Copeland; variety E: Hokuiku 39; variety F: Ryofu; variety G: Lofty Nijo), and 7 beers were produced using the same materials except for malt. The protein Z7 concentrations in the Congress worts produced from seeds of the 7 barley varieties used as a material for beer and the NIBEM values of the 7 beers were measured, and the correlation between the protein Z7 concentration in Congress wort and NIBEM value was examined.

The Congress worts were produced as follows. The malt of each barley variety produced by the procedure described in Example 1 was ground, and 200 mL or water was added to the ground malt (50 g). After being held at 45°C for 30 minutes, the mixture was heated to 70°C at a rate of 1°C/min. When the temperature reached 70°C, 100 mL of water was added, after which the mixture was held for 1 hour. Water was then added to a total weight of 450 g, and the wort obtained by filtration was treated as Congress wort.

The beer brewing and NIBEM value measurement were performed by the procedures described in Example 1. The protein Z7 concentration in Congress wort was measured by appropriately diluting each liquid and performing ELISA with an anti-protein Z7 antibody.

Table 2 shows the protein Z7 concentrations in the Congress worts produced from seeds of the 7 barley varieties (varieties A to G) and the NIBEM values of the 7 beers brewed using seeds of the 7 barley varieties as a material.

**[Table 2]**

| Barley variety. | Protein Z7 concentration in Congress wort (µg/mL) | NIBEM value (sec) |
|---|---|---|
| Variety A | 41.0 | 234 |
| Variety B | 12.7 | 265 |
| Variety C | 44.7 | 243 |
| Variety D | 25.8 | 247 |
| Variety E | 37.8 | 226 |
| Variety F | 27.9 | 251 |
| Variety G | 50.4 | 229 |

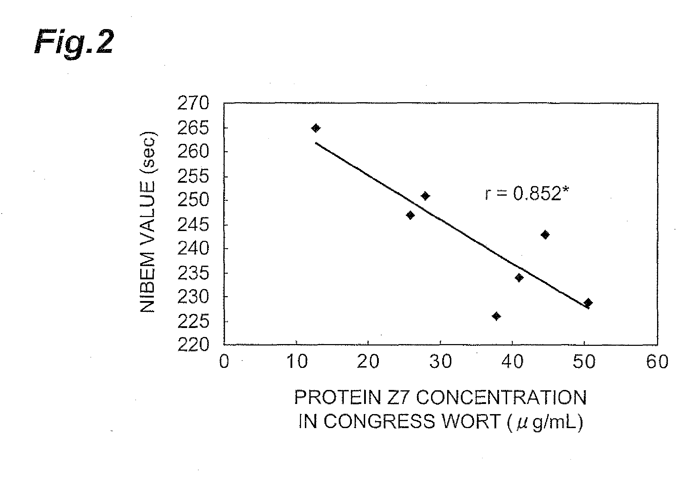

Fig. 2 is a graph showing the result of simple regression analysis performed using the NIBEM value as the response variable and the protein Z7 concentration in Congress wort as the explanatory variable. A single asterisk in the graph indicates significance at the 5% level.

A statistically significant correlation was found between the NIBEM value and protein Z7 concentration in Congress wort. The simple regression equation was: NIBEM value = 273.05 + (-0.9006 × protein Z7 concentration) (r = 0.852).

The obtained results demonstrate that the protein Z7 concentration in Congress wort can be used as an index having a negative correlation with the foam stability of a fermented malt beverage produced using the wort as a material, to determine the foam stability of the fermented malt beverage, and that measurement of the protein Z7 concentration in wort allows estimation of the NIBEM value of the fermented malt beverage. Also, the results demonstrate that measurement of the protein Z7 concentration in wort allows selection of a barley that improves the foam stability of a fermented malt beverage.

### [Example 3: Correlation between the protein Z7 concentration in beer and the NIBEM value]

Malts were produced from seeds of 7 barley varieties (variety A: Haruna Nijo; variety B: CDC Kendall; variety C: Amagi Nijo; variety D: CDC Copeland; variety E: Hokuiku 39; variety F: Ryofu; variety G: Lofty Nijo), and 7 beers were produced using the same materials except for malt. The protein Z7 concentrations in the 7 beers and the NIBEM values of the 7 beers were measured, and the correlation between the protein Z7 concentration in beer and NIBEM value was examined.

The beer brewing and NIBEM value measurement were performed by the procedures described in Example 1. The protein Z7 concentration in beer was measured by appropriately diluting each liquid and performing ELISA with an anti-protein Z7 antibody.

Table 3 shows the protein Z7 concentrations in the 7 beers brewed using seeds of the 7 barley varieties (varieties A to G) as a material and the NIBEM values of the 7 beers.

**[Table 3]**

| Barley variety | Protein Z7 concentration in beer (µg/mL) | NIBEM value (sec) |
|---|---|---|
| Variety A | 13.99 | 234 |
| Variety B | 1.83 | 265 |
| Variety C | 5.99 | 243 |
| Variety D | 8.76 | 247 |
| Variety E | 12.21 | 226 |
| Variety F | 5.38 | 251 |
| Variety G | 12.55 | 229 |

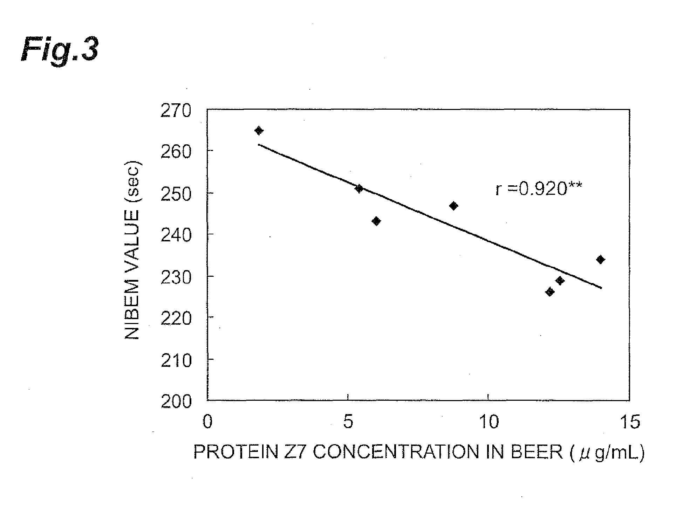

Fig. 3 is a graph showing the result of simple regression analysis performed using the NIBEM value as the response variable and the protein Z7 concentration in beer as the explanatory variable. A double asterisk in the graph indicates significance at the 1% level.

A statistically significant correlation was found between the NIBEM value and protein Z7 concentration in beer. The simple regression equation was: NIBEM value = 266.50 + (-2.8089 × protein Z7 concentration) (r = 0.920).

The obtained results demonstrate that the protein Z7 concentration in beer can be used as an index having a negative correlation with the foam stability of the beer, to determine the foam stability of the fermented malt beverage, and that measurement of the protein Z7 concentration in beer allows estimation of the NIBEM value of the fermented malt beverage. Also, the results demonstrate that measurement of the protein Z7 concentration in beer allows selection of a barley that improves the foam stability of a fermented malt beverage.

## Claims

1. A method for determination of the foam stability of a fermented malt beverage, wherein the concentration of protein Z7 (M_{Z7}) in seeds of a barley which is used as a material for the fermented malt beverage, a malt obtained from the seeds, a wort obtained from the malt, a fermented malt beverage obtained by fermenting the wort, or a pre-fermentation or fermenting material solution of the fermented malt beverage, is used as an index having a negative correlation with the foam stability of the fermented malt beverage, to determine the foam stability of the fermented malt beverage.

2. The determination method according to claim 1, wherein the value of the formula: a - b × M_{Z7} [where a is a positive or negative number or 0, and b is a positive number] is used as an index having a positive correlation with the foam stability of the fermented malt beverage, to determine the foam stability of the fermented malt beverage.

3. The determination method according to claim 2, wherein a in the formula: a - b × M_{Z7} is 0.

4. The determination method according to claim 2, wherein a and b in the formula: a - b × M_{Z7} are the simple regression coefficients of a predictive simple regression equation for the NIBEM value of the fermented malt beverage as a function of M_{Z7}.

5. A marker for determination of the foam stability of a fermented malt beverage, the marker being composed of protein Z7.

6. A method for selection of a barley that improves the foam stability of a fermented malt beverage, wherein protein Z7 is used as a selection marker, the method comprising:
a measurement step in which the concentration of protein Z7 in seeds of a barley, a malt obtained from the seeds, a wort obtained from the malt, a fermented malt beverage obtained by fermenting the wort, or a pre-fermentation or fermenting material solution of the fermented malt beverage, is measured, and
a judgment step in which, if the concentration is lower than a prescribed reference value, the barley is judged to be a barley that improves the foam stability of a fermented malt beverage.

7. The selection method according to claim 6, wherein the measurement step comprises:
an extraction step in which protein is extracted from seeds of the barley, a malt obtained from the seeds, a wort obtained from the malt, a fermented malt beverage obtained by fermenting the wort, or a pre-fermentation or fermenting material solution of the fermented malt beverage, and
a quantitation step in which an anti-protein Z7 antibody is used to quantitate protein Z7 in the protein.

8. A method for selection of a barley that improves the foam stability of a fermented malt beverage, wherein protein Z7 is used as a selection marker, the method comprising:
an extraction step in which RNA is extracted from seeds of a barley or a malt obtained from the seeds,
a quantitation step in which protein Z7 mRNA expressed in the seeds or malt is quantitated, and
a judgment step in which, if the protein Z7 mRNA expression level is lower than a prescribed reference value, the barley is judged to be a barley that improves the foam stability of a fermented malt beverage.

9. A marker for selection of a barley that improves the foam stability of a fermented malt beverage, the marker being composed of protein Z7.
